Europäisches Patentamt

European Patent Office

Office européen des brevets

(11) Veröffentlichungsnummer: **0 294 684**
**A2**

(12) **EUROPÄISCHE PATENTANMELDUNG**

(21) Anmeldenummer: 88108653.2

(22) Anmeldetag: 31.05.88

(51) Int. Cl.⁴: **C07C 47/04 , C07C 45/51**

(30) Priorität: 06.06.87 DE 3719055
06.04.88 DE 3811509

(43) Veröffentlichungstag der Anmeldung:
14.12.88 Patentblatt 88/50

(84) Benannte Vertragsstaaten:
**BE CH DE ES FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Renken, Albert, Prof. Dr.**
**46, chemin des Charmilles**
**CH-1025 Saint-Sulpice(CH)**
Erfinder: **Meyer, Andreas**
**5, place de la Cathédrale**
**CH-1005 Lausanne(CH)**

(54) **Verfahren zur Herstellung von Formaldehyd.**

㉗ Die Erfindung betrifft ein Verfahren zur Herstellung von Formaldehyd aus Methanol durch Dehydrierung in Gegenwart eines Katalysators bei erhöhter Temperatur. Die Umsetzung wird in Gegenwart eines metallisches Natrium oder mindestens eine Natriumverbindung enthaltenden Katalysators bei einer Temperatur von 300° C bis 800° C durchgeführt.

EP 0 294 684 A2

## Verfahren zur Herstellung von Formaldehyd

Die vorliegende Erfindung betrifft ein Verfahren zur Herstellung von Formaldehyd durch Dehydrierung von Methanol in Gegenwart eines Katalysators bei erhöhter Temperatur

Es sind mehrere Verfahren zur Herstellung von Formaldehyd aus Methanol bekannt. In der Technik ist die Oxydation von Methanol zu Formaldehyd üblich

$$CH_3OH + \tfrac{1}{2} O_2 \rightarrow CH_2O + H_2O$$

die an Eisen- und Molybdänoxyd enthaltenden Katalysatoren bei 350°C bis 450°C durchgeführt wird. Ebenfalls üblich ist die oxydative Dehydrierung von Methanol zu Formaldehyd

$$CH_3OH \rightleftharpoons CH_2O + H_2$$

$$H_2 + \tfrac{1}{2} O_2 \rightarrow H_2O$$

an Silberkatalysatoren bei 600 bis 720°C. Beide Verfahren werden beschrieben in Ullmanns Encycl. der techn. Chemie, Band 11, S. 693 - 694, 4. Auflage, 1976, Verlag Chemie Weinheim.

Nach diesen Verfahren erhält man eine wässerige Formaldehydlösung. Die Lagerung und der Transport solcher Lösungen sind jedoch schwierig, da Niederschläge von Paraformaldehyd und entsprechende Ablagerungen und Verstopfungen auftreten, wenn nicht Stabilisatoren und erhöhte Lagertemperaturen verwendet werden. Erhöhte Lagertemperatur beeinflußt aber in unerwünschter Weise die Produktqualität durch Bildung von Ameisensäure.

Aus diesen Gründen wurden schon Verfahren zur Herstellung von im wesentlichen wasserfreien methanolischen Formaldehydlösungen vorgeschlagen. Die DE-OS 25 25 174 beschreibt einen Kupfer, Zink und Schwefel enthaltenden Katalysator. Die US-PS 4,054,609 schildet einen Katalysator, der Kupfer, Zink und Selen enthält.

Weitere Katalysatoren enthalten Zink und/oder Indium (Europäische Patentschrift 0 130 068), Silber (US-PS 2,953,602), bzw. Silber, Kupfer und Silicium (US-PS 2,939,883). Alle diese Katalysatoren ermöglichen keine wirtschaftliche Herstellung von Formaldehyd durch Dehydrierung von Methanol.

Gegenstand der vorliegenden Erfindung ist ein Verfahren zur Herstellung von Formaldehyd aus Methanol durch Dehydrierung in Gegenwart eines Katalysators bei erhöhter Temperatur, dadurch gekennzeichnet, daß die Umsetzung in Gegenwart eines metallisches Natrium oder mindestens eine Natriumverbindung enthaltenden Katalysators bei einer Temperatur von 300°C bis 800°C durchgeführt wird.

Die Reaktion kann durch die Gleichung

$$CH_3OH \rightleftharpoons CH_2O + H_2$$

beschrieben werden.

Das Verfahren liefert überraschenderweise Formaldehyd mit guter Selektivität und Ausbeute.

Das Natrium kann als Metall oder Verbindung oder in beiden Formen eingesetzt werden. Vorzugsweise wird es als Verbindung eingesetzt, was jedoch nicht ausschließt, daß es während der Reaktion zumindest teilweise als Metall vorliegt. Zur Herstellung des Katalysators eignen sich vor allem das Oxid, das Nitrat, das Carbonat, das Hydrogencarbonat, das Sulfat, das Acetat, das Oxalat, das Molybdat und die Borate. Vorzugsweise verwendet man neutrale oder basische Salze, insbesondere das Carbonat, das Molybdat oder das Tetraborat.

Der erfindungsgemässe Katalysator enthält vorzugsweise zusätzlich zu metallischem Natrium bzw. der oder den Natriumverbindungen noch bis zu 3.0 Grammatome Indium pro Grammatom Natrium, insbesondere 0.001 bis 3.0 Grammatome Indium pro Grammatom Natrium. Das Indium kann dabei während der Reaktion als Metall und/oder als Metallverbindung vorliegen; es wird im allgemeinen als Metallverbindung aufgebracht, die während der Reaktion oder vorab ganz oder teilweise zum Metall reduziert werden kann.

Zur Herstellung des Katalysators eignen sich vor allem Indiumverbindungen, wie etwa das Oxid, das Molybdat, das Nitrat, das Carbonat, das Hydrogencarbonat, das Acetat, das Oxalat, das Sulfat, das Sulfid oder eines der Halogenide. Vorzugsweise verwendet man das Oxid, das Molybdat, das Nitrat, das Sulfat, das Sulfid oder eines der Halogenide, insbesondere das Nitrat.

Der Katalysator kann in Form von Kugeln oder Stäbchen sowie in jeder anderen Gestalt verwendet werden. Der Katalysator kann allein oder mit einem inerten Träger wie zum Beispiel Siliciumdioxyd eingesetzt werden. Der Ausgangskatalysator kann unmittelbar eingesetzt werden oder zuvor thermisch und/oder chemisch aktiviert werden, zum Beispiel in reduzierender Atmosphäre mit Wasserstoff.

Die Dehydrierung des Methanols kann bei Unterdruck, Normaldruck oder Überdruck, zum Beispiel 10 bar, durchgeführt werden. Ein Bereich von etwa 0.1 bis 10 bar ist besonders geeignet, aber der Druck ist unkritisch. Bevorzugt wird Normaldruck. Das erfindungsgemässe Verfahren kann diskontinuierlich oder kontinuierlich durchgeführt werden, wobei letzteres bevorzugt ist. Die Temperatur beträgt im allgemeinen 300°C bis 800°C, bevorzugt 500°C bis 700°C. Es werden vorzugsweise 0,1 bis 10 kg Methanol pro Stunde und pro Liter Katalysator umgesetzt. Das Methanol kann rein oder verdünnt mit einem zusätzlichen Gas wie Stickstoff oder Wasserstoff am Katalysator zu Formaldehyd dehydriert werden.

Die Erfindung wird an folgenden Beispielen erläutert, ohne darauf beschränkt zu sein. Die in den Beispielen angegebenen Meßgrössen wurden wie folgt berechnet.

$$\text{Umsatzgrad} = \frac{\text{umgesetztes Methanol (mol)}}{\text{zugeführtes Methanol (mol)}}$$

$$\text{Selektivität} = \frac{\text{gebildeter Formaldehyd (mol)}}{\text{umgesetztes Methanol (mol)}}$$

Als Nebenreaktion tritt lediglich eine gewisse Bildung von CO und $H_2$ aus Methanol ein.

Beispiel 1

Natriumkarbonat wurde stufenweise im Wasserstoffstrom kalziniert. Die Aufheizungsgeschwindigkeit betrug 5° C/min. Bei 100° C, 500° C, 600° C und 650° C wurde die Temperatur jeweils drei Stunden belassen. Die Endtemperatur von 700° C wurde fünf Stunden gehalten. Von dem so erhaltenen Katalysator wurden 2 g in ein Quarzrohr von 10 mm Innendurchmesser gefüllt. Stündlich wurden 0,16 l eines Stickstoff-Methanol-Gemisches mit 17% Methanolgehalt durch die Katalysatorschüttung geleitet. Bei 700° C wurde das Methanol zu 20% umgesetzt. Formaldehyd wurde mit 65% Selektivität erhalten.

Beispiel 2

Zwei Gramm Natriumtetraborat wurden in ein Quarzrohr von 10 mm Innendurchmesser gegeben. Bei einem Zulauf von 0,171 mol/h (22% Methanol, Rest Stickstoff) und 700° C Reaktionstemperatur bildete sich Formaldehyd mit einer Selektivität von 52%. Das Methanol wurde zu 21% umgesetzt.

Beispiel 3

Indiumnitrat und Natriumtetraborat wurden so vermischt, daß auf ein Mol Indium 19 Mole Natrium kamen. Das Gemisch wurde im Stickstoffstrom aktiviert. Bis 500° C wurde mit 5° C/min aufgeheizt und drei Stunden belassen. Danach wurde mit 1° C/min bis auf 700° C aufgeheizt. Der so erhaltene Katalysator wurde klassiert und die 40 bis 80 Mikrometerfraktion in ein Quarzrohr von 10 mm Innendurchmesser gegeben, so daß eine Schütthöhe von 10 mm erhalten wurde. Durch diese Schüttung wurden stündlich 0,154 mol eines Stickstoff-Methanol-Gemisches geleitet, das 13% Methanol enthielt. Formaldehyd wurde bei 700° C in 31%iger Ausbeute erhalten. (Ausbeute = Gebildeter Formaldehyd/Zugeführtes Methanol, beides in Mol gerechnet).

Beispiel 4

Ein Natrium und Indium enthaltender Katalysator wurde wie in Beispiel 3 beschrieben hergestellt. Dabei wurden die Ausgangsmengen jedoch so abgeändert, daß auf 1 Teil Indium 5,7 Teile Natrium kamen. Von der 100 bis 400 Mikrometerfraktion wurde eine 60 mm Schüttung in einem Quarzrohr von 10 mm Innendurchmesser verwendet. Bei einem Zulauf von 0,154 mol/h und 700° C Reaktionstemperatur wurde Formaldehyd mit 56,5% Ausbeute erhalten. Der Zulauf bestand zu 13% aus Methanol und zu 87% aus Stickstoff.

Beispiel 5

Natriumphosphat wurde drei Stunden bei 350° C und fünf Stunden bei 500° C in Luft kalziniert. Von dem so erhaltenen Katalysator wurden zwei Gramm in ein Quarzrohr von 10 mm Innendurchmesser gegeben und stündlich 0.171 mol Zulauf (22% Methanol, Rest Stickstoff) durch die Schüttung geleitet. Bei 700° C bildeten sich 8% Formaldehyd. Das Methanol wurde zu 18% umgesetzt.

Beispiel 6

In ein Quarzrohr von 10 mm Innendurchmesser wurden 2 g Natriumsulfat gegeben. Bei einem Zulauf von 0,171 mol/h (22% Methanol, Rest Stickstoff) und 700° C Reaktionstemperatur wurden 39,5% des eingesetzten Methanols umgesetzt. Formaldehyd bildete sich mit 16,5% Ausbeute und 41,8% Selektivität.

Beispiel 7

Natriummolybdat wurde bei 110° C getrocknet und drei Stunden bei 300° C sowie fünf Stunden bei 500° C aktiviert. Durch eine Schüttung von 17 mm Höhe in einem Quarzrohr von 10 mm Innendurchmesser wurden 0,161 mol/h eines Gemisches aus 17% Methanol und 83 % Stickstoff geleitet. Bei 700° C wurde das Methanol zu 30% umgesetzt. Formaldehyd bildete sich mit 14% Ausbeute und 47% Selektivität.

Beispiel 8

Natriumhydrogenkarbonat wurde bei 250° C in Natriumkarbonat umgewandelt. Von dem so erhaltenen Natriumkarbonat wurde die 40 - 80 Mikrometerfraction in ein Quarzrohr von 10 mm Innendurchmesser gegeben, so daß eine Schütthöhe von 10 mm erhalten wurde. Durch diese Schüttung wurden stündlich 0,136 mol eines Stickstoff-Methanol-Gemisches geleitet, das 14% Methanol enthielt. Bei 650° C wurde das Methanol zu 56% umgesetzt und Formaldehyd mit 65% Selektivität erhalten.

Beispiel 9

99 Teile Natriumkarbonat und 1 Teil Indiumoxyd wurden vermischt. Dabei wurde das Natriumkarbonat wie in Beispiel 8 beschrieben hergestellt. Von dem so erhaltenen Katalysator wurde die 40 - 80 Mikrometerfraktion in ein Quarzrohr von 10 mm Innendurchmesser gegeben, so daß eine Schütthöhe von 10 mm erhalten wurde. Durch diese Schüttung wurden stündlich 0,136 mol eines Methanol-Stickstoff-Gemisches geleitet, das 14% Methanol enthielt. Bei 650° C wurde das Methanol zu 40% umgesetzt und Formaldehyd mit 78% Selektivität gebildet.

Beispiel 10

Natriummolybdatdihydrat wurde bei 100° C und 0,2 bar Absolutdruck entwässert. Die 40 - 100 Mikrometerfraktion wurde mit Indiumoxyd der Korngröße 40 - 80 Mikrometer vermischt, wobei auf 1 Teil Indiumoxyd 5,6 Teile Natriummolybdat kamen. Der so erhaltene Katalysator wurde in ein Quarzrohr von 10 mm Innendurchmesser gegeben, so daß eine Schütthöhe von 10 mm erreicht wurde. Durch diese Schüttung wurden bei 700° C stündlich 0,133 mol eines Methanol-Stickstoff-Gemisches geleitet, das 12% Methanol enthielt. Formaldehyd wurde mit 46% Ausbeute und 74% Selektivität erhalten.

**Ansprüche**

1. Verfahren zur Herstellung von Formaldehyd aus Methanol durch Dehydrierung in Gegenwart eines Katalysators bei erhöhter Temperatur, dadurch gekennzeichnet, daß die Umsetzung in Gegenwart eines metallisches Natrium oder mindestens eine Natriumverbindung enthaltenden Katalysators bei einer Temperatur von 300° C bis 800° C durchgeführt wird.

2. Verfahren nach Anspruch 1, dadurch gekennzeichnet, daß der Katalysator zusätzlich bis zu 3.0 Grammatome Indium pro Grammatom Natrium enthält.

3. Verfahren nach Anspruch 1, dadurch gekennzeichnet, daß der Katalysator zusätzlich 0.001 bis 3.0 Grammatome Indium pro Grammatom Natrium enthält.

4. Verfahren nach Anspruch 1 oder 2, dadurch gekennzeichnet, daß man bei einer Temperatur von 500°C bis 700°C arbeitet.